Europäisches Patentamt

European Patent Office

Office européen des brevets

(19)

(11) Veröffentlichungsnummer: **0 039 885**
**B1**

(12) **EUROPÄISCHE PATENTSCHRIFT**

(45) Veröffentlichungstag der Patentschrift:
16.05.84

(51) Int. Cl.³: **G 01 N 33/86**

(21) Anmeldenummer: 81103395.0

(22) Anmeldetag: 05.05.81

(54) Verfahren und Reagens zum Nachweis von Fibrinmonomer.

(30) Priorität: 08.05.80 DE 3017707

(43) Veröffentlichungstag der Anmeldung:
18.11.81 Patentblatt 81/46

(45) Bekanntmachung des Hinweises auf die Patenterteilung:
16.05.84 Patentblatt 84/20

(84) Benannte Vertragsstaaten:
AT BE CH DE FR GB IT LI LU NL SE

(56) Entgegenhaltungen:
DE - A - 2 134 928
DE - A - 2 709 311
DE - A - 2 746 100
CHEMICAL ABSTRACTS, Band 82, Nr. 3, 20. Januar 1975, Seite 213, Nr. 13297v Columbus, Ohio, U.S.A. U.P. MÜLLER et al.: "Sensitive latex agglutination method for detecting soluble fibrins in plasma"
CHEMICAL ABSTRACTS, Band 81, Nr. 11, 16. September 1974, Seite 218, Nr. 60468r Columbus, Ohio, U.S.A. A.E. FINKELSTEIN et al.: "New nonimmunological method of measuring fibrinogen-fibrin degradation products"
CHEMICAL ABSTRACTS, Band 90, Nr. 13, 26. März 1979, Seite 230, Nr. 99591m Columbus, Ohio, U.S.A. J.E. RUTSTEIN et al.: "Rheumatoid factor interference with the latex agglutination test for fibrin degradation products"

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Becker, Udo, Dr., Schmaedelstrasse 17,
D-8000 München 60 (DE)
Erfinder: Roeschlau, Peter, Dr., Schönegertstrasse 4,
D-8124 Seeshaupt (DE)

(74) Vertreter: Weickmann, Heinrich, Dipl.-Ing. et al,
Patentanwälte Dipl.-Ing. H. Weickmann Dipl.-Phys.Dr. K., Fincke Dipl.-Ing. F.A. Weickmann Dipl.-Chem. B. Huber
Dr.-Ing. H. Liska Möhlstrasse 22,
D-8000 München 86 (DE)

Verfahren und Reagens zum Nachweis von Fibrinmonomer

Die Erfindung betrifft ein Verfahren und ein Reagens zum Nachweis und zur Bestimmung von Fibrinmonomer (FS) in Blutplasma und anderen biologischen Flüssigkeiten.

Der frühen Erkennung einer Thrombosebereitschaft kommt eine große diagnostische Bedeutung zu. Hierzu müssen latent ablaufende Gerinnungsvorgänge, die noch nicht zu einem äußerlich sichtbaren klinischen Bild führen, erkannt werden. Bisher gibt es keine empfindlichen oder keine klinisch praktikablen Methoden, um auf einfache Weise Aktivierungsprodukte des Gerinnungssystems zu einem frühen Stadium nachzuweisen. Einer der unmittelbarsten Indikatoren für den Ablauf von Gerinnungsvorgängen ist das FS, das durch die Einwirkung von Thrombin auf Fibrinogen entsteht. Geringe Mengen von FS werden durch den im Blut vorhandenen Überschuß an Fibrinogen stabilisiert und löslich erhalten, ohne daß es zur Ablagerung im Gefäßsystem kommt. Aufgabe eines diagnostischen Verfahrens ist es daher geringe Mengen dieses in löslicher Form zirkulierenden FS in Anwesenheit eines Überschusses an Fibrinogen nachzuweisen und zu bestimmen. Da sich FS von Fibrinogen weder durch das Molekulargewicht noch durch die Antigenstruktur unterscheiden läßt, sind die üblichen biochemischen Nachweisverfahren nicht anwendbar. Folgende Methoden zum Nachweis von FS sind bereits bekannt:

1. Fällungsverfahren, die die gegenüber Fibrinogen größere Neigung des FS zur Präzipitation ausnützen, z. B. Äthanoltest nach Breen und Tullis (Annals of Internal Medicine, Bd. 69, Seite 1197—1206 [1968] oder Protaminsulfattest nach Niewiarowski und Gurewich (J. Lab. Clin. Med., Bd. 77, Seite 665—676 [1971]). Zur besseren Erkennung der ausflockenden Fibrinfäden wurden kolloidale gefärbte Partikel, z. B. Rußteilchen, eingesetzt (DE-A-25 28 381). Neben der Gefahr unspezifischer Präzipitation haben diese Methoden den Nachteil, unempfindlich zu sein und nur qualitative Hinweise zu geben.

2. Gelausschlußchromatographie (Fletcher und Alkjaersig in: Recent-Advances in Thrombosis, L. Poller Editor, London 1973, Livingstone-Churchill-Verlag) und Affinitätschromatographie mit Fibrinagarose (Heene und Matthias, Thromb. Research, Bd. 2, 137—154 [1973]). Diese Methoden basieren auf der Eigenschaft con FS, mit Fibrinogen hochmolekulare Komplexe zu bilden, bzw. der Selbstassoziation von FS. Sie sind nicht als klinische Routinemethoden anzusehen, weil sie nicht praktikabel und auch nicht ausreichend empfindlich sind.

3. Bekannt ist weiter ein auf der Selbstassoziation von FS beruhendes Verfahren (Largo, Heller und Straub in: Blood, Vol. 47, Nr. 6 [Juni 1976]), bei welchem zuerst mit FS beladene Erythrozyten hergestellt werden, welche dann in Anwesenheit des gelösten FS in einer zu untersuchenden Probe agglutinieren. Dieses Verfahren hat sich als klinischer Test ebenfalls nicht durchgesetzt, insbesondere wegen ungenügender Empfindlichkeit.

4. Einbau von 14 C Glycinester (Kisker und Rush, J. Clin. Invest. 50, Seite 2235, 1971). Mit Hilfe von Faktor XIII kann radioaktiv markierter 14-C-Glycinester spezifisch in FS eingebaut werden. Das Verfahren ist empfindlich, aber wenig praktikabel und erfordert die üblichen umständlichen Maßnahmen und aufwendigen Vorrichtungen beim Umgang mit Radioaktivität.

Der Erfindung liegt die Aufgabe zugrunde, die Nachteile der bekannten Verfahren zu beseitigen und ein Verfahren der genannten Art zu schaffen, welches einfach und rasch durchführbar ist, keiner aufwendigen Vorrichtungen bedarf und sich sowohl zur qualitativen, halbquantitativen als auch zur quantitativen Bestimmung eignet.

Es wurde nun überraschenderweise gefunden, daß Fibrinmonomer (FS) im Gegensatz zu Fibrinogen und anderen Plasmaproteinen eine hohe Affinität zu hydrophoben Latexpartikeln zeigt.

Das erfindungsgemäße Verfahren zum Nachweis und zur Bestimmung von Fibrinmonomer in biologischen Flüssigkeiten ist dadurch gekennzeichnet, daß man die Probeflüssigkeit mit Latex inkubiert und die Agglutination bestimmt.

Vorzugsweise werden erfindungsgemäß hydrophobe Latices, deren Partikel oder Tröpfchen einen mittleren Durchmesser von 0,05 bis 1,5, besonders bevorzugt von 0,5 bis 1,2 μm bei Messung im Aggregationsmeßgerät, von 0,07 bis 0,1 bei Messung im Photometer, aufweisen, verwendet. Jedoch eignen sich auch Latices oder Tröpfchen mit einem außerhalb dieser Bereiche liegenden Durchmesser. So wurden auch mit Latexpartikeln von 0,03 und von 10 μm noch brauchbare Ergebnisse erhalten. Unter Latex werden dabei im Rahmen der Erfindung Emulsionen und sonstige Dispersionen von natürlichen oder synthetischen Kautschukpartikeln, insbesondere die in Wasser dispergierten Polymerisat- oder Mischpolymerisat-Teilchen, beispielsweise aus Styrol oder Styrol und Butadien verstanden. Für die Erfindung wesentlich ist, daß die Partikel oder Tröpfchen eine hydrophobe Oberfläche aufweisen.

Die Inkubation der Probeflüssigkeit mit der Latex wird zweckmäßig bei erhöhter Temperatur durchgeführt. Bevorzugt wird eine Temperatur zwischen 30 und 40° C, da hierbei die Umsetzung am raschesten abläuft. Es können aber auch niedrigere Temperaturen bei entsprechend verlängerter Inkubationsdauer oder gegebenenfalls auch noch etwas darüber liegende Temperaturen angewendet werden. Die Vorinkubationszeit bei einer Temperatur von 37° C beträgt zweckmäßig 5 bis 15 Minuten. Bei einer Unterschreitung dieses Bereichs wird die Reproduzierbarkeit in Frage gestellt, eine Überschreitung ergibt keinen Vorteil.

Die Umsetzung wird zweckmäßig in gepufferter Lösung vorgenommen, wobei sich pH-Werte zwischen 7,0 und 9,0 als besonders geeignet erwiesen haben. Bevorzugt werden pH-Werte zwischen 7,7 und 8,7. Zur Einstellung des gewünschten pH-Wertbereichs eignen sich beliebige, im genannten Bereich wirksame Puffersubstanzen. Bevorzugt wird ein Glycin/Kochsalz-Puffer. Die Pufferkonzentration liegt zweckmäßig zwischen 0,05 und 0,5 M.

Die qualitative Durchführung des erfindungsgemäßen Verfahrens wird zweckmäßig so durchgeführt, daß man die zu untersuchende Probe, beispielsweise Plasma, mit einer geeigneten wäßrigen Suspension von Latexpartikeln inkubiert, wobei vorzugsweise die oben angegebenen Temperatur- und Zeitbereiche eingehalten werden.

Anschließend wird das Reaktionsgemisch vorsichtig geschüttelt oder auf eine Testplatte, die in einzelne Felder unterteilt sein kann, welche durchsichtig oder auch zur besseren Beobachtung dunkel grundiert sein kann, aufgetropft und die Testplatte kreisförmig bewegt. Bei Anwesenheit von FS in der Untersuchungsflüssigkeit kommt es zu einer Verklumpung der Partikel, welche makroskopisch sichtbar ist. Zur besseren Sichtbarmachung der Reaktion kann die Teilchensuspension auch zentrifugiert werden, wobei die agglutinierten Teilchen schneller und leichter sedimentieren als nichtagglutinierte. Vorteilhafterweise kann die Reaktion auch in Mikrotiterplatten durchgeführt werden, welche zur Beschleunigung der Ablesung zusätzlich zentrifugiert werden können.

Zur halbquantitativen Durchführung des Verfahrens stellt man Serienverdünnungen her und vergleicht diese mit einer Probe, welche FS in bekannter Menge enthält. Hierbei wird zweckmäßig, die höchste, noch zu einer sichtbaren Agglutination führende Verdünnungsstufe mit der Nachweisgrenze der Reaktion multipliziert. Diese Nachweisgrenze läßt sich durch Verwendung eines Standards mit bekanntem FS-Gehalt in der gleichen Weise durch Ausverdünnung bestimmen. Quantitativ läßt sich das Verfahren bei Anwendung eines Aggregationsmeßgeräts oder eines Photometers durchführen.

Unter Fibrin, welches durch das erfindungsgemäße Verfahren bestimmt werden kann, wird das sogenannte lösliche Fibrin verstanden, also eine Zwischenform des Fibrins, bei welcher sich das Fibrinmonomer im eigentlichen Sinne bereits zu Fibrillen zusammengelagert hat, an der Oberfläche aber entweder noch echtes Fibrinmonomer trägt oder jedenfalls sich wie Fibrinmonomer verhält. Dieses lösliche Fibrin führt ebenfalls zur gleichen Agglutination und wird daher ebenfalls erfaßt.

Ein weiterer Gegenstand der Erfindung ist ein Reagens zum Nachweis und zur Bestimmung von Fibrinmonomer in biologischen Flüssigkeiten, welches gekennzeichnet ist durch einen Gehalt an hydrophoben Latexpartikeln.

Die Latex weist zweckmäßig einen Partikelgehalt zwischen 0,1 und 10%, vorzugsweise zwischen 0,5 und 5% auf. Latex und Probelösung werden in Volumenverhältnissen zwischen etwa 1 : 10 und 10 : 1 gemischt. Verwendet man eine 1%ige Suspension, so beträgt das Mischungsverhältnis vorzugsweise 2 : 1 bis 1 : 2.

Die Erfindung eignet sich auch zur indirekten Bestimmung von Fibrinogen. Hierbei wird alles Fibrinogen der Probe zuerst in bekannter Weise in Fibrinmonomer umgewandelt. Die Umwandlung erfolgt mit einem geeigneten Enzym. Geeignete Enzyme sind beispielsweise Thrombin oder Schlangengifte, wie Batroxobin. Stellt man in einer Parallelprobe weiter den Gehalt an Fibrinmonomer fest, so ergibt sich aus der Differenz zwischen dem ermittelten FS-Gehalt in der Vergleichsprobe und der mit dem Enzym behandelten Probe der Fibrinogengehalt. Vorzugsweise wird bei dieser Ausführungsform der Erfindung die Umwandlung des Fibrinogens in Fibrinmonomer in Anwesenheit der Latexpartikel durchgeführt.

Die folgenden Beispiele erläutern die Erfindung weiter.

### Beispiel 1

A. Einer gesunden Person wird unter Vorlage von 1 mg/ml EDTA und 0,1 mg/ml Aprotinin Blut entnommen und 10 Minuten bei 2000 g zentrifugiert. 1 ml des Überstands von Blutplasma wird auf 37° C gebracht und mit 0,05 Einheiten Thrombin (T opostasin, Roche) vermischt. Aliquote von 0,2 ml werden zu bestimmten Zeiten entnommen und in vorbereitete Röhrchen pipettiert, welche 5 Einheiten Heparin (Liquemin, Firma Roche) und 0,2 Einheiten Hirudin (Sigma) enthalten (Abstoppreaktion). Als Zeitpunkte der Entnahme von Aliquoten wurden gewählt: sofort nach Zugabe des Thrombins, 30 Sekunden, 1 Minute, 2 Minuten und 5 Minuten. Man erhält so unterschiedliche Mengen von FS enthaltende Proben, die für die weiteren Untersuchungen bei Raumtemperatur aufbewahrt werden.

B. Verdünnungen der Proben werden in einem Glycin-NaCl-Puffer, welcher 0,25 mol Glycin und 0,9% NaCl enthält und mit 1 n NaOH auf einen pH-Wert von 8,2 eingestellt wurde, hergestellt. Eine Polystyrollatex der Firma Serva/Heidelberg in Form einer 10%igen Suspension mit einem mittleren Teilchendurchmesser von 0,8 μm wird mit demselben Puffer 1 : 10 verdünnt. Je 0,05 ml der Latex und 0,05 ml der in geeigneter Weise vorverdünnten Plasmaprobe werden gemischt und 10 Minuten bei 37° C inkubiert. Der Inhalt der Röhrchen wird auf die einzelnen voneinander getrennten Felder einer dunkel lackierten Kunststoffplatte aufgebracht, die Platte 2 Minuten lang vorsichtig rotiert und anschließend die Verklumpung der Latexteilchen registriert. Es zeigt sich, daß mit zunehmender Dauer der Thrombineinwirkung auf das Plasma die Intensität der Verklumpung zunimmt. Durch Einsatz steigender Verdünnungen kann für jede Probe diejenige Verdünnung angegeben werden, bei der gerade noch eine deutliche Agglutination auftritt. Die Ergebnisse sind in Tabelle 1 aufgeführt.

Tabelle 1

| Behandlungsdauer mit Thrombin (min) | Probe | Titer |
| --- | --- | --- |
| 0 | Plasma | 1 : 16 |
| 0,5 | Plasma | 1 : 64 |
| 1 | Plasma | 1 : 1600 |
| 2 | Plasma | 1 : 6400 |
| 5 | Plasma | 1 : 25 600 |
| 0 | Puffer | negativ |
| 0 | Serum, unverdünnt | negativ |

Eine mit Puffer oder Serum durchgeführte Kontrolle ergibt keinerlei Agglutination.

### Beispiel 2

Die nach Beispiel 1 hergestellten FS-haltigen Proben werden in Glycin-NaCl-haltigem Puffer verdünnt, dem zusätzlich 1% Rinderserumalbumin zugesetzt wurde. Die Ergebnisse sind in Tabelle 2 aufgeführt.

Tabelle 2

| Behandlungsdauer mit Thrombin (min) | Probe | Titer |
| --- | --- | --- |
| 0 | Plasma | 1 : 8 |
| 0,5 | Plasma | 1 : 32 |
| 1 | Plasma | 1 : 128 |
| 2 | Plasma | 1 : 256 |
| 5 | Plasma | 1 : 512 |
| 0 | Puffer | negativ |
| 0 | Serum | negativ |

### Beispiel 3

Nach der Vorschrift von Mahn et al., Thromb. Res. 14, Seite 651—663 (1979) wird eine Lösung von FS in 3 Mol Harnstoff, 50 mmol Tris, 5 mmol EDTA, 0,01% Aprotinin, pH 7,4, hergestellt und der Gehalt an FS nach Verdünnung in 0,1 n NaOH über die optische Dichte bei 280 nm bestimmt. Nach Beispiel 1 gewonnenes Plasma wird mit Glycin/NaCl, pH 8,2, 1 : 8 verdünnt. Durch Zugabe steigender Mengen der harnstoffhaltigen FS-Lösung werden Proben von definiertem Gehalt an FS herstellt; und zwar 0,5, 10 und 50 mg/ml, wobei Volumenausgleich durch FS-freie Harnstofflösung erfolgt. Die Proben werden entsprechend Beispiel 2 mit der Latexsuspension inkubiert und die letzte, jeweils noch zu einer Agglutinationsreaktion führende Verdünnungsstufe festgestellt. Die Ergebnisse aus zwei unabhängigen Bestimmungen sind in Tabelle 3 zusammengestellt.

Tabelle 3

| FS-Konzentration $\mu$g/ml | Titer | Nachweisgrenze $\mu$g/ml |
| --- | --- | --- |
| 5 | 1 : 4 | 1,3 |
| 10 | 1 : 10 | 1,0 |
| 50 | 1 : 40 | 1,3 |

Aus den jeweiligen Grenzverdünnungen, die gerade noch zu einer positiven Agglutination führen, läßt sich die Nachweisgrenze für FS durch Multiplikation der jeweiligen FS-Konzentration mit der Verdünnung berechnen. Im Mittel ergibt sich eine Nachweisgrenze von ca. 1,2 $\mu$mg/ml.

### Beispiel 4

Eine Fibrinogenlösung mit einem Gehalt von 500 mg/dl und menschliches Citratplasma mit einem Fibrinogengehalt von 180 mg/dl wird mit Glycin-NaCl-Puffer, pH 8,2, welcher 1% Rinderserumalbumin enthält, 1 : 100 verdünnt. Davon ausgehend werden weitere Serienverdünnungen 1 : 2, 1 : 4 etc. in demselben Puffer hergestellt und je 0,1 ml davon in Röhrchen pipettiert. Zu jedem Röhrchen werden nacheinander je 0,1 ml einer mit 0,9%iger NaCl-Lösung 1 : 10 vorverdünnte Lösungen von Batroxobin (Reptilase-Reagenz®, Boehringer Mannheim) und 0,1 ml der nach Beispiel 1 hergestellten Latex pipettiert und 10 Minuten bei 37°C inkubiert. Anschließend wird durch Auftropfen auf eine Testplatte der Agglutinationstiter bestimmt. Man findet für die Fibrinogenlösung 1 : 16, für das Plasma 1 : 8, was bei der Berücksichtigung der Vorverdünnung 1 : 100 einer Verdünnung 1 : 1600 bzw. 1 : 800 entspricht. Daraus errechnet sich eine Nachweisgrenze für Fibrinogen von ca. 2,7 mg/ml.

### Beispiel 5

Verdünnungen eines nach Beispiel 1 hergestellten, mit Thrombin 5 Minuten lang behandelten Plasmas in rinderserumalbuminhaltigem Glycin-NaCl-Puffer wird mit der Latexsuspension über verschiedene Zeiten bei 37°C inkubiert und dann der Agglutinationstiter bestimmt. Es wird gefunden, daß die Empfindlichkeit aber nach einer Inkubationsdauer von 10 Minuten nicht mehr ansteigt.

### Beispiel 6

Entsprechend Beispiel 3 wird eine FS-Lösung in 3 M Harnstoff hergestellt. Eine Fibrinogen-Lösung in Glycin/NaCl-Puffer, pH 8,2, mit einem Fibrinogengehalt von 2 mg/ml wird in 5 · 1 ml aufgeteilt, auf 37°C gebracht und die Aliquote mit steigenden Mengen der FS-Lösung versetzt, so daß Gehalte von 10, 20, 30, 40 und 50 µg/ml entstehen.

Ein handelsübliches Aggregometer, Fa. Braun/Melsungen, mit Schreiber, wie es üblicherweise für die Messung der Thrombozyten-Aggregation verwendet wird, wird nachträglich mit einer regulierbaren Rühreinrichtung ausgestattet.

Es wird der folgende Ansatz durchgeführt: Filter 546 nm, 37°C, Rührgeschwindigkeit 500 UpM. 1 ml Glycin/NaCl-Puffer wird vorgelegt und Temperaturanpassung auf 37°C abgewartet. Dazu werden 0,1 ml einer 1%igen Latexsuspension in Glycin/NaCl-Puffer gegeben und am Photometer die Extinktion 1,9 eingestellt. Es werden je 50 µl der oben beschriebenen FS-haltigen Fibrinogenlösungen zugegeben. Die Verklumpung der Latexpartikel führt zu einer Aufhellung in der Lösung, die vom Schreiber aufgezeichnet wird. Der Versuch wird nacheinander mit allen FS-Konzentrationen durchgeführt, einschließlich der Fibrinogen-Lösung ohne FS-Zusatz und einem Pufferwert. Es zeigt sich, daß die verwendete Fibrinogen-Lösung bereits einen gewissen Gehalt an FS enthält, da auch ohne FS-Zusatz bereits eine Trübungsaufhellung festgestellt wird. Die maximalen Extinktionsabnahmen des Aggregometers werden gegen die FS-Konzentrationen aufgetragen, wobei sich eine lineare Beziehung ergibt, aus der die Reaktionsempfindlichkeit als die Steigung abzulesen ist. Man findet 14 mE/µg FS. Diese Steigung kann außerdem zur Ermittlung des in der verwendeten Fibrinogen-Lösung bereits enthaltenen FS verwendet werden: Man findet 51 µg/ml, 2,6% des Fibrinogengehalts.

### Beispiel 7

In einem handelsüblichen Photometer, versehen mit Küvettenwechselautomatik für 6 Küvetten und Schreiber, wird folgender Ansatz durchgeführt: Filter 405 nm, 37°C, 1 cm Halbmikroküvetten.

In die 6 Küvetten werden je 1 ml Glycin/NaCl-Serumalbumin-Puffer, pH 8,2, eingefüllt und Temperaturangleichung auf 37°C abgewartet. In die Küvetten 1 bis 6 werden je 100 µl der nach Beispiel 1 hergestellten thrombinbehandelten Plasmaproben gegeben und gemischt. Dann wird zu jedem Ansatz 0,1 ml einer in Glycin/NaCl-Puffer, pH 8,2, auf 0,5% vorverdünnten Latexsuspension (Serva, mittlerer Teilchendurchmesser 0,085 µm) gefügt, gemischt und gleichzeitig der Küvettenwechsler sowie der Schreiber gestartet. Die Ansätze zeigen einen linearen Extinktionsanstieg in Abhängigkeit von der Behandlungsdauer mit Thrombin, welcher zahlenmäßig in Tabelle 4 aufgeführt ist.

Tabelle 4

| Behandlungsdauer mit Thrombin (sec) | Extinktionsanstieg ($\Delta$E/min) |
|---|---|
| 0 | 0 |
| 30 | 0,011 |
| 60 | 0,049 |
| 90 | 0.090 |
| 180 | 0,130 |
| 300 | 0,144 |

### Patentansprüche

1. Verfahren zum Nachweis und zur Bestimmung von Fibrinmonomer in biologischen Flüssigkeiten, dadurch gekennzeichnet, daß man die Probeflüssigkeit mit hydrophobem Latex inkubiert und die Agglutination bestimmt.

2. Verfahren nach Anspruch 1, dadurch gekennzeichnet, daß man eine hydrophobe Latex mit einem mittleren Teilchendurchmesser von 0,05 bis 1,5 µm verwendet.

3. Verfahren nach Anspruch 1 oder 2, dadurch gekennzeichnet, daß die Inkubation bei erhöhter Temperatur durchgeführt wird.

4. Verfahren nach Anspruch 3, dadurch gekennzeichnet, daß man bei einer Temperatur von 30 bis 40°C 5 bis 15 Minuten inkubiert.

5. Verfahren nach einem der vorhergehenden Ansprüche, dadurch gekennzeichnet, daß man in gepufferter Lösung bei einem pH-Wert zwischen 7,0 und 9,0 inkubiert.

6. Verfahren nach einem der vorhergehenden Ansprüche, dadurch gekennzeichnet, daß man zur qualitativen Durchführung die Probe nach der Inkubation mit dem Latex auf eine dunkelfarbige Testplatte bringt und die Agglutination feststellt.

7. Verfahren nach einem der Ansprüche 1 bis 5, dadurch gekennzeichnet, daß man zur quanti-

tativen Durchführung Serienverdünnungen der Probeflüssigkeit herstellt und mit einem Standard von bekanntem Gehalt an FS vergleicht.

8. Verfahren nach einem der Ansprüche 1 bis 5, dadurch gekennzeichnet, daß man zur quantitativen Durchführung mit Hilfe optischer Verfahren die durch die Agglutination verursachte Trübungsänderung bestimmt.

9. Verfahren nach einem der vorhergehenden Ansprüche, dadurch gekennzeichnet, daß man das in der Probeflüssigkeit vorhandene Fibrinogen zunächst in an sich bekannter Weise mit Thrombin oder einem Schlangengiftenzym in FS überführt.

10. Reagens zum Nachweis und zur Bestimmung von Fibrin oder Fibrinmonomer in biologischen Flüssigkeiten, gekennzeichnet durch einen Gehalt an hydrophobem Latex.

11. Reagens nach Anspruch 10, dadurch gekennzeichnet, daß es hydrophobe Latexpartikel mit einem Durchmesser von 0,05 bis 1,5 µm enthält.

12. Reagens nach einem der Ansprüche 10 oder 11, dadurch gekennzeichnet, daß es eine Polystyrollatex enthält.

## Claims

1. Process for the detection and for the determination of fibrin monomer in biological fluids, characterised in that one incubates the sample fluid with hydrophobic latex and determines the agglutination.

2. Process according to claim 1, characterized in that one uses a hydrophobic latex with an average particle diameter of 0,05 to 1,5 µm.

3. Process according to claim 1 or 2, characterized in that the incubation is carried out at an elevated temperature.

4. Process according to claim 3, characterized in that one incubates at a temperature of 30 to 40° C. for 5 to 15 minutes.

5. Process according to one of the preceding claims, characterized in that one incubates in buffered solution at a pH between 7,0 and 9,0.

6. Process according to one of the preceding claims, characterized in that, for the qualitative carrying out, one applies the sample, after the incubation with the latex, to a dark-coloured test plate and ascertains the agglutination.

7. Process according to one of claims 1 to 5, characterised in that, for the quantitative carrying out, one prepares serial dilutions of the sample fluid and compares with a standard with a known content of FS.

8. Process according to one of claims 1 to 5, characterized in that, for the quantitative carrying out with the help of optical processes, one determines the change of turbidity brought about by the agglutination.

9. Process according to one of the preceding claims, characterized in that one first converts the fibrinogen present in the sample fluid in per se known manner into FS with thrombin or a snake venom enzyme.

10. Reagent for the detection and for the determination of fibrin or fibrin monomer in biological fluids, characterized by a content of hydrophobic latex.

11. Reagent according to claim 10, characteriSed in that it contains hydrophobic latex particles with a diameter of 0,05 to 1,5 µm.

12. Reagent according to claim 10 or 11, characteried in that it contains a polystyrene latex.

## Revendications

1. Procédé pur la détection et pour le dosage de la fibrine monomère dans des liquides biologiques, caractérisé en ce que l'on incube le liquide échantillon avec du latex hydrophobe et on détermine l'agglutination.

2. Procédé selon la revendication 1, caractérisé en ce que l'on utilise un latex hydrophobe à un diamètre de particules moyen de 0,05 à 1,5 µm.

3. Procédé selon la revendication 1 ou 2, caractérisé en ce que l'incubation est effectuée à chaud.

4. Procédé selon la revendication 3, caractérisé en ce que l'on incube à une température de 30 à 40° C pendant 5 à 15 minutes.

5. Procédé selon l'une des revendications qui prédèdent, caractérisé en ce que l'on incube en solution tamponnée à une valeur de pH entre 7,0 et 9,0.

6. Procédé selon l'une des revendications qui précèdent, caractérisé en ce que, pour la mise en oeuvre qualitative, on porte l'échantillon, après incubation avec le latex, sur une plaque pour essais de couleur sombre et on observe l'agglutination.

7. Procédé selon l'une des revendications 1 à 5, caractérisé en ce que, pour la mise en oeuvre quantitative, on prépare des dilutions en série du liquide échantillon et on compare avec un étalon à teneur connue en FS.

8. Procédé selon l'une des revendications 1 à 5, caractérisé en ce que, pour la mise en oeuvre quantitative à l'aide d'un procédé optique, on détermine la variation du trouble causée par l'agglutination.

9. Procédé selon l'une des revendications qui précèdent, caractérisé en ce que l'on convertit d'abord le fibrinogène présent dans le liquide échantillon en FS de manière connue en soi à l'aide de thrombine ou d'une enzyme de venin de serpent.

10. Réactif pour la détection et le dosage de la fibrine ou de la fibrine monomère dans des liquides biologiques, caractérisé par une teneur en latex hydrophobe.

11. Réactif selon la revendication 10, caractérisé en ce qu'il contient des particules hydrophobes de latex à un diamètre de 0,05 à 1,5 µm.

12. Réactif selon l'une des revendications 10 ou 11, caractérisé en ce qu'il contient un latex polystyrène.